# EUROPEAN PATENT SPECIFICATION

(11) **EP 2 367 506 B1**
(45) Date of publication and mention of the grant of the patent: **25.06.2014**
(21) Application number: 09822541.0
(22) Date of filing: 20.10.2009
(51) Int. Cl.: A61F 2/36, A61F 2/46, A61B 19/00

(54) **PATIENT MATCHED HIP SYSTEM**
AUF EINEN PATIENTEN ABGESTIMMTES HÜFTSYSTEM
SYSTÈME DE HANCHE ACCORDÉ AU PATIENT

(30) Priority: 22.10.2008 US 255935
(43) Date of publication of application: 28.09.2011
(73) Proprietor: Biomet Manufacturing Corp., Warsaw, IN 46581 (US)
(72) Inventor: WHITE, John, R., Winona Lake IN 46590 (US)
(74) Representative: Giles, Ashley Simon
(86) International application number: PCT/US2009/061293
(87) International publication number: WO 2010/048156

(56) References cited:
- EP-A1- 1 574 183
- US-A1- 2008 021 567
- US-B1- 6 205 411

## Description

### FIELD

This invention relates to a method of selecting a patient matched hip joint prosthesis. The closest prior art is document US 6205411, which defines the preamble of claim 1.

### BACKGROUND

The statements in this section merely provide background information related to the present disclosure and may not constitute prior art.

A hip joint can be replaced with a hip joint prosthesis to reduce pain due to arthritis, deterioration, deformation, and the like. In some embodiments, the hip joint prosthesis includes femoral components (i.e., components that are implanted in the femur) and pelvic components (i.e., components that are implanted in the pelvis), and the femoral components are movably coupled to the pelvic components to replicate the mechanics of the anatomical hip joint.

The femoral components of the hip joint prosthesis can include a stem member that extends into the intramedullary canal in the femur, a head member that movably couples to the pelvic components, and a neck member that couples the stem and head members.

In many cases, a custom designed, substantially monolithic hip joint prosthesis is designed and manufactured for an individual patient to provide a desired hip center of rotation, stability, and range of motion. However, because each patient is sufficiently different, the dimensions of such a custom designed prosthesis are unlikely to be adequate for another patient. Thus, designing and manufacturing individual custom hip joint prosthetics can be quite costly. Also, since these custom prosthetics may require custom instrumentation for implantation, the cost can be further increased. Moreover, the surgical procedure may be different for each custom prosthetic, possibly resulting in increased surgical time.

In order to reduce these costs and surgical time, modular hip joint prosthesis systems have been proposed. These systems can include a plurality of standardized stem members, a plurality of standardized head members, and a plurality of standardized neck members. Each of the stem members, head members, and neck members differ dimensionally. Also, each of the members can removably attach to each other to form a wide variety of hip joint prostheses. Thus, a stem member, head member, and neck member from each set can be individually chosen and assembled according to the individual patient. In other words, a different prosthesis can be assembled from these members for each individual patient. Because these members (and the associated instruments and surgical procedures) are standardized, costs and surgical time can be significantly reduced.

In order to plan for surgery and to choose the modular components that are appropriate for an individual patient, doctors can use dimensional images of the anatomical hip with overlays of different modular components. Other methods involve measuring certain anatomical features of the hip joint and choosing the components based on those measurements. However, a surgeon may realize during surgery that the selected components do not provide adequate range of motion and/or stability of the joint. Also, the standard, modular components may not allow for the desired dimensional adjustments to the prosthesis. Thus, choosing and building an appropriate hip joint prosthesis for a patient and planning for surgery can be difficult and imprecise.

### SUMMARY

The invention is defined in claim 1.

The method includes imaging a hip joint anatomy of a patient, creating an electronic model of the hip joint anatomy, and identifying a plurality of first parameters of the hip joint anatomy of the patient. The first parameters include a neck length of a femur of the patient, a neck angle of the femur, an amount of anteversion of the femur, and a vector relative to a center of a head of the femur and an axis of a neck of the femur. Furthermore, the method includes selecting a standard modular neck member from a set of different standard modular neck members to substantially match the first parameters of the hip joint anatomy of the patient. In addition, the method includes performing a hip joint mechanical dynamics analysis using the selected standard modular neck member. The hip joint mechanical dynamics analysis includes performing a range of motion analysis and performing a stability analysis of the hip joint using the selected standard modular neck member. Additionally, the method includes choosing for implantation either the selected standard modular neck member, a different one of the standard modular neck members, or a custom designed neck member based on the hip joint mechanical dynamics analysis. Also, the method includes building a provisional neck member corresponding in shape to the neck member chosen for implantation.

Further areas of applicability will become apparent from the description provided herein. It should be understood that the description and specific examples are intended for purposes of illustration only and are not intended to limit the scope of the present disclosure.

### DRAWINGS

The drawings described herein are for illustration purposes only and are not intended to limit the scope of the present disclosure in any way.
FIG. 1 is an exploded perspective view of a modular patient matched hip joint prosthesis;
FIG. 2 is a modular schematic diagram showing a system and method of selecting components for the patient matched hip joint prosthesis;
FIG. 3 is a schematic diagram showing a tool for selecting the components of the modular patient matched hip joint prosthesis;
FIG. 4 is a flowchart of a method of selecting components of a modular patient matched hip joint prosthesis;
FIG. 5 is a coronal view of a hip joint anatomy model of a patient and a set of components available for the modular patient matched hip joint prosthesis;
FIG. 6 is a sagittal view of the hip joint anatomy model and the set of components available for the modular patient matched hip joint prosthesis; and
FIG. 7 is an inferior view of the hip joint anatomy model and the set of components available for the modular patient matched hip joint prosthesis

### DETAILED DESCRIPTION

The following description is merely exemplary in nature and is not intended to limit the present disclosure, application, or uses. It should be understood that throughout the drawings, corresponding reference numerals indicate like or corresponding parts and features.

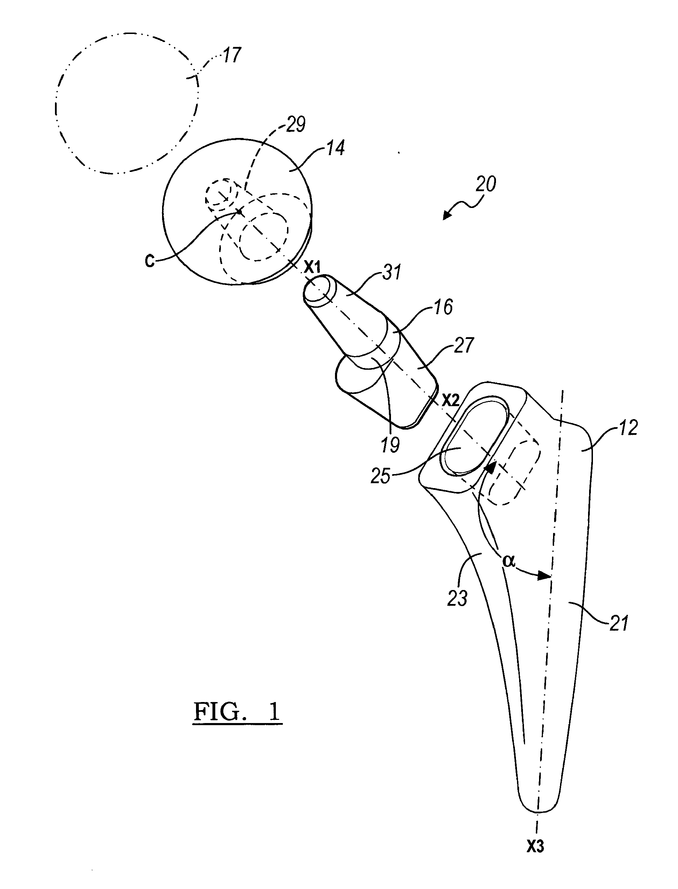

Referring initially to FIG. 1, an exemplary modular patient matched hip joint prosthesis 20 is illustrated. In the example shown, the prosthesis 20 is a modular system that includes a stem member 12, a head member 14, and a neck member 16. The members 12, 14, 16 can be made from any suitable material, such as titanium, cobalt chrome, ceramic, diamond, etc.

The neck member 16 is interposed between the stem member 12 and the head member 14. The neck member 16 removably couples the stem member 12 and the head member 14 and will be discussed in greater detail below.

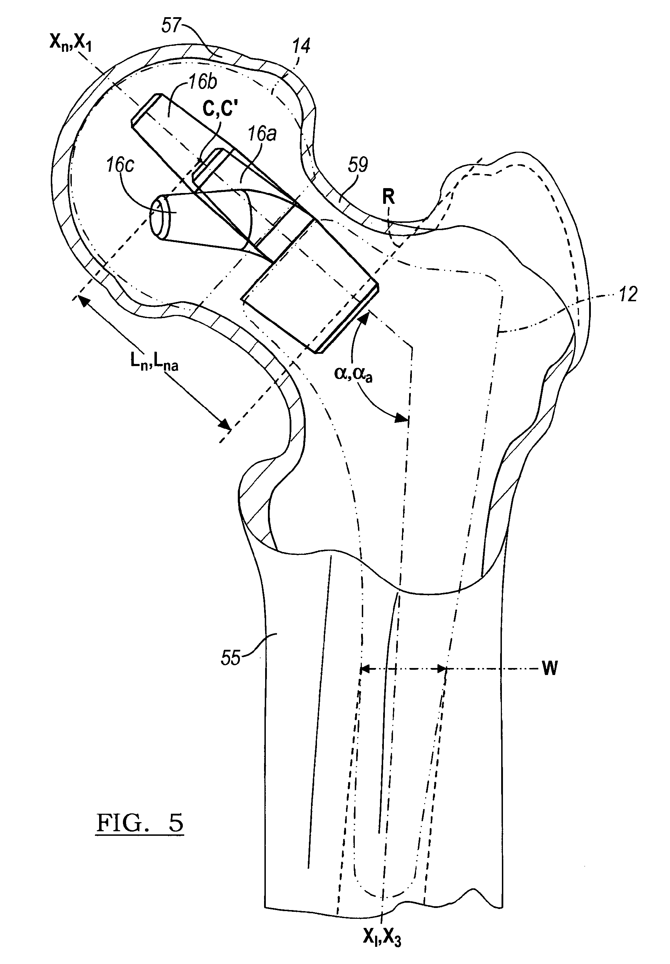

The stem member 12 defines an axis X3, and is adapted to couple to a resected femur within the intramedullary canal (FIG. 5). In some examples, the stem member 12 includes a male connecting portion 21, such as a male tapered connecting portion 21 for securing the stem member 12 within the intramedullary canal. The stem member 12 also includes an intermediate portion 23 that couples to the neck member 16 as will be discussed. In some examples, the stem member 12 includes a female tapered opening 25 that receives a male tapered end 27 of the neck member 16. In some embodiments, the male tapered end 27 is oblong in cross sectional shape, and the female tapered opening 25 is correspondingly oblong such that the stem member 12 can removably couple to the neck member 16 via a taper lock coupling. Also, in some examples, the intermediate portion 23 of the stem member 12 is at least partially disposed superior to a resection line R of the femur (FIG. 5).

Also, the head member 14 defines an axis X1 and is adapted to be positioned within an acetabulum of a pelvis (not specifically shown) for articulation therein substantially about the center C of the head member 14. More specifically, a prosthetic acetabular cup 17 can be coupled to the pelvis within the accetabulum, and the head member 14 can moveably couple to the prosthetic acetabular cup 17 for articulation within the accetabulum. In other examples, the head member 14 is moveably coupled to the natural anatomy of the patient's pelvis for articulation therein. In some examples, the head member 14 includes a female tapered opening 29 that receives a male tapered end 31 of the neck member 16. In some examples, the male tapered end 31 is circular in cross section, and the female tapered opening 29 is correspondingly circular in cross section such that the head member 14 can removably couple to the neck member 16 via a taper lock coupling.

The neck member 16 also includes an intermediate portion 19 between the ends 27, 31 of the neck member 16. Furthermore, the neck member 16 defines an axis X2. In some examples, the axis X2 is substantially straight and is collinear with the axis X1 of the head member 14. In other examples, the axis X2 of the neck member 16 has a positive angle or is curved as will be discussed.

It will be appreciated that the neck member 16 can affect an angular displacement between the axis X1 of the head member 14 and the axis X3 of the stem member 12. This angular displacement between the axes X1, X3 can be referred to as a neck angle α (FIG. 1). It will be appreciated that the neck angle α could be of any suitable value.

Furthermore, it will also be appreciated that the neck member 16 can also affect a distance Lₙ between the center C of the head member 14 and the resection line R (FIG. 5). This distance can be referred to as a neck length Lₙ. It will be appreciated that the neck angle α and neck length Lₙ can affect the mechanical characteristics of the hip joint prosthesis 20 and can affect the stability and range of motion of the hip joint prosthesis 20.

Still further, it will be appreciated that the patient matched hip joint prosthesis 20 illustrated in FIG. 1 is merely one example thereof, and the prosthesis 20 could have any suitable shape. Moreover, the prosthesis 20 could include any suitable number of members, and one or more of the stem, head, and neck members 12, 14, 16 could be integrally attached together so as to be monolithic. For instance, the neck and head members 14, 16 could be integrally attached (i.e., monolithic) and could removably attach to a modular stem member 12. Also, the stem and neck members 12, 16 could be integrally attached (i.e., monolithic) and could removably attach to a modular head member 14.

Furthermore, it will be appreciated that the taper lock coupling shown in FIG. 1 is merely one example of the coupling between the neck member 16, stem member 12, and head member 14, and the members 12, 14, 16 could be coupled via any other appropriate means. Moreover, the neck member 16 could include a respective female portion of a taper coupling on either end, and the head member 14 and/or stem member 12 could include a respective male portion of a taper coupling to couple to the neck member 16.

Moreover, it will be appreciated that the prosthesis 20 is modular, and as such, the members 12, 14, 16 are replaceable with different members 12, 14, 16 such that the prosthesis 10 is highly adaptable to different patients' anatomy. As will be discussed, while each of the members 12, 14, 16 are adapted to fit a particular patient's anatomy, the dimensions of the neck member 16 are particularly important for properly providing a desired center of rotation of the head member 14, sufficient stability, and adequate range of motion of the artificial hip joint.

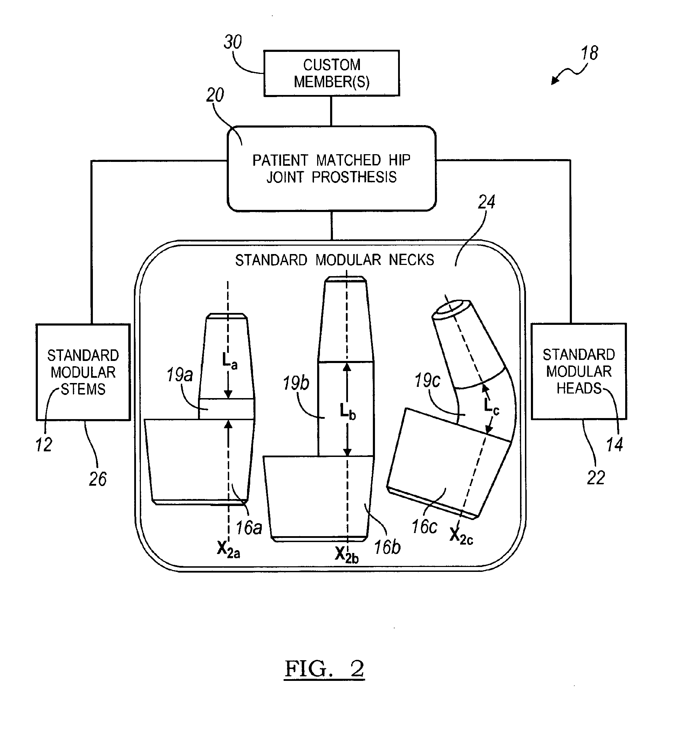

Referring now to FIG. 2, a prosthesis system or kit 18 is schematically illustrated. As illustrated, components of the modular patient matched hip joint prosthesis 20 are selected from the system 18. The system 18 includes a set 24 or plurality of standard, modular neck members 16a, 16b, 16c. The system 18 also includes a set 22 or plurality of standard, modular head members 14. Furthermore, the system 18 includes a set 26 or plurality of standard, modular stem members 12.

As shown, the neck members 16a, 16b, 16c within the set 24 each has differing dimensional features. For instance, the respective intermediate portion 19a, 19b, 19c of each neck member 16a, 16b, 16c has a different length Lₐ, L_{b}, L_{c}, which affects the neck length Lₙ of the prosthesis 20 as described above. Moreover, the axes X₂ₐ, X_{2b} of the neck members 16a, 16b are each substantially straight, while the axis X_{2c} of the neck member 16c is angled, and these features will affect the neck angle α of the prosthesis 20 as described above. As will be discussed, a surgeon can select between the neck members 16a, 16b, 16c to adjust the prosthesis 20 in various ways. For instance, the neck members 16a, 16b, 16c can be chosen to adjust the neck angle α and/or the neck length Lₙ. It will be appreciated that, although only three neck members 16a, 16b, 16c are illustrated for exemplary purposes, there can be any number of neck members 16a, 16b, 16c within the set 24. Furthermore, the neck members 16a, 16b, 16c can have any suitable dimension and any additional feature, including a longitudinal axis that is curved.

It will be appreciated that, similar to the set 24 of modular neck members 16a, 16b, 16c, the set 22 can include a plurality of head members 14 of differing dimensions. For instance, the set 22 can include a plurality of head members 14 of differing size (e.g., differing radii) and/or different material (e.g., titanium and ceramic).

Moreover, the set 26 can include a plurality of stem members 12 having differing dimensions. For instance, the set 26 can include a plurality of stem members 14 of differing body size (e.g., differing width) as well as different axial shape (e.g., curved and straight axes).

Each of the neck members 16a, 16b, 16c can removably attach to any one of the stem members 12 within the set 26 as well as any one of the head members 14 within the set 22. Thus, the neck members 16a, 16b, 16c in the set 24 are interchangeable with the stem members 12 in the set 26 and with the head members 14 in the set 22.

Thus, the patient matched hip joint prosthesis 20 can be assembled from one of the neck members 16a, 16b, 16c in the set 22, one of the head members 14 in the set 24, and one of the stem members 12 in the set 26. Alternatively, as will be discussed, the patient matched hip joint prosthesis 20 can be a custom designed member, generally indicated at 30.

By assembling the prosthesis 20 from the sets 22, 24, 26 of standard, modular members, the prosthesis 20 can sufficiently match the patient's anatomy, provide sufficient range of motion, provide sufficient stability, and yet reduce costs and surgery time. However, if an adequate prosthesis 20 cannot be assembled from the sets 22, 24, 26, a custom designed member 30 can be provided. The custom designed member 30 can replace each of the standard, modular stem, head, and neck members 12, 14, 16 of the sets 22, 24, 26, and the custom designed member 30 can be monolithic. In other examples, the hip joint prosthesis 20 is assembled from one or more of the standard, modular members of the sets 22, 24, 26 as well as a custom designed member 30. For instance, the prosthesis 20 could include a standard stem member 12 from the set 26, a standard head member 14 from the set 24, and a custom neck member 30.

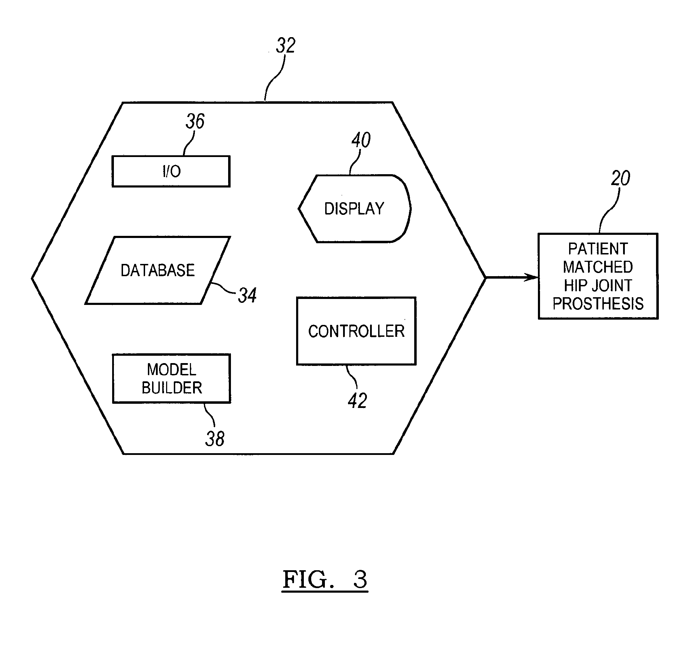

Referring now to FIG. 3, a computerized tool 32 or system is schematically illustrated. The tool 32 is used to select, design, and virtually assemble the patient matched hip joint prosthesis 20 in a manner to be described. The tool 32 includes a database 34 (e.g., a memory device) in which the data representing the standard size members in each of sets 22, 24, 26 are stored. The tool 32 further includes an interface 36 (e.g., I/O) for sending/receiving data to/from an external source. The interface 36 can be of any suitable type, and can include a keyboard, mouse, wireless connectors, USB ports, and the like. The tool 32 further includes a model builder 38 for building anatomical models of the patient's hip joint and a model of the hip joint prosthesis 20. Furthermore, the tool 32 includes a display 40 for displaying to a medical professional the models generated by the model builder 38. In addition, the tool 32 includes a controller 42 for controlling the functions of the database 34, the interface 36, the model builder 38, and the display 40. As will be explained, the tool 32 can aid the medical professional in selecting, designing, and virtually assembling the components of the patient matched hip joint prosthesis 20. This process can be performed in a substantially automatic manner, based on automatically detected measurements and inputs, the process can be performed with a higher degree of personal input from a medical professional, or the process can be performed using a combination of the tool's computational ability and the medical professional's knowledge and expertise. It will be appreciated that the tool 32 can be used in the planning stages of surgery. Thus, the computerized tool 32 can sufficiently increase the efficiency and accuracy of the surgical procedure.

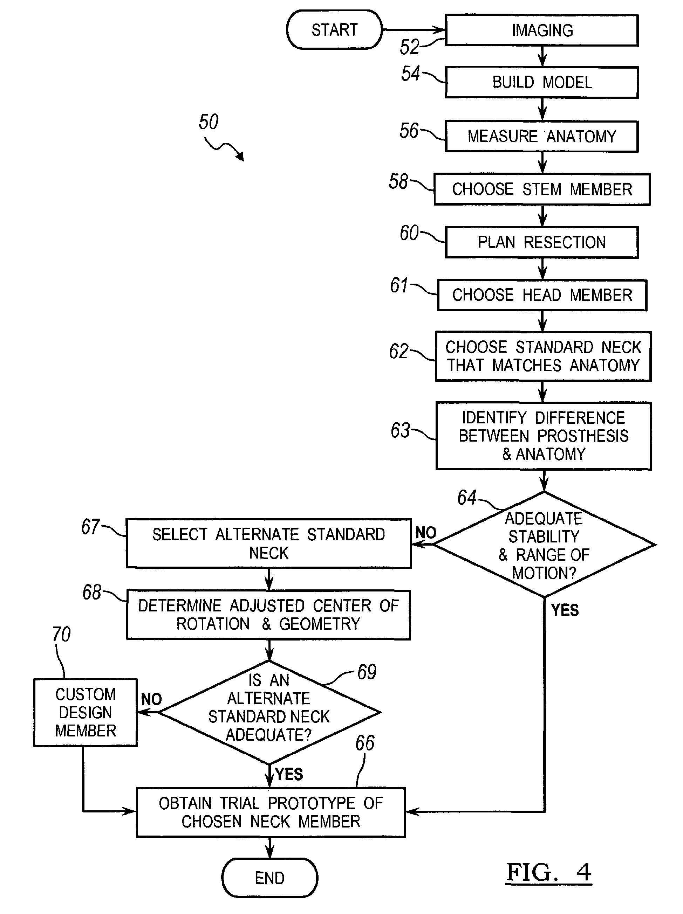

Referring now to FIG. 4, a method 50 of selecting and designing the patient matched hip joint prosthesis 20 is illustrated. It will be appreciated that the method 50 can be employed using the computerized tool 32 illustrated in FIG. 3. The method 50 begins at block 52, which involves imaging the hip joint anatomy of the patient. For instance, in some embodiments, block 52 involves performing a CT scan, an MRI, and/or a fluoroscopic scan.

Then, in block 54, the imaging data is transmitted via the interface 36 to the tool 32, and the model builder 38 creates an electronic model of the hip joint anatomy. In some embodiments, a three-dimensional electronic model of the hip joint anatomy is generated. However, in some embodiments, a two-dimensional model is generated. FIGS. 5, 6, and 7 represent the model generated in block 54. As shown, a femur 55 of the patient is illustrated in the model, and the femur 55 includes a head 57 and a neck 59.

It will be appreciated that the model can include skeletal features and can include muscle tissue as well. For instance, in some embodiments, the model generated in block 54 can include muscle attachments the surgeon plans on leaving undisturbed during the procedure.

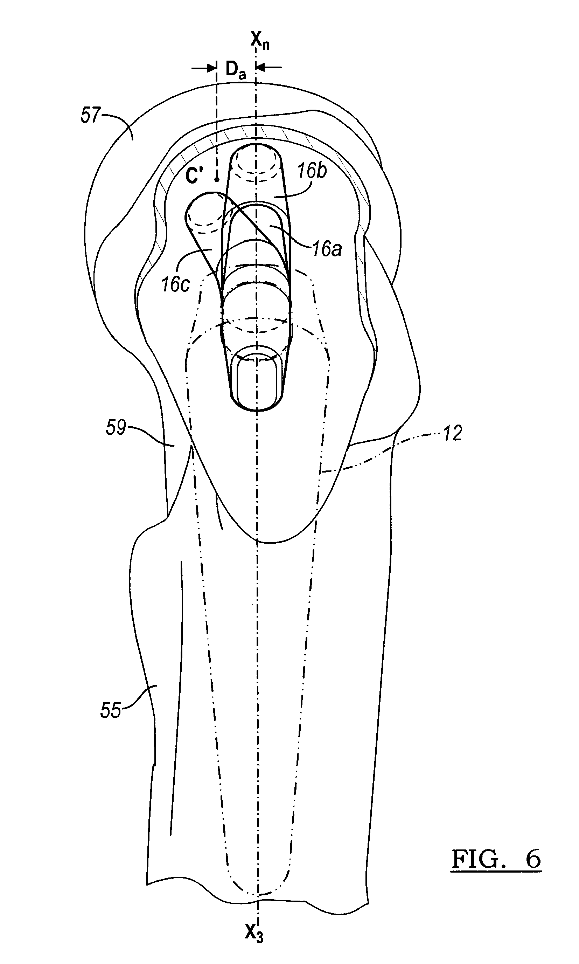

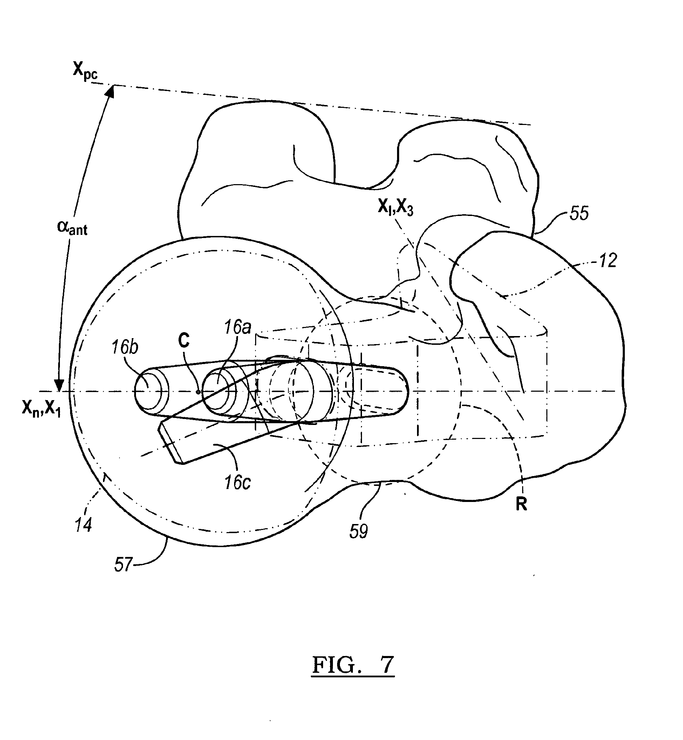

Using the model, a plurality of predetermined parameters (e.g., anatomical dimensions) can be identified and measured in block 56. In some embodiments, the tool 32 is used to identify a neck length Lₙₐ of the femur 55 (FIG. 5). Also, in some embodiments, a neck angle αₐ (FIG. 5) between an axis Xₙ of the neck 59 and a longitudinal axis Xₗ of the femur 55 is identified. Also, in some embodiments, an amount of anteversion αₐₙₜ (FIG. 7) between the axis Xₙ of the neck 59 and the posterior condyle axis X_{pc} of the femur 55 is identified. Furthermore, in some embodiments, a vector Dₐ (FIG. 6) relative to the center C' of the head 57 and the axis Xₙ of the neck 59 of the femur 55 is identified (i.e., the amount of distance and the direction between the center C' and the axis Xₙ). Other anatomical features can also be measured via the model, such as the length of the leg, the width W of the intramedullary canal (FIG. 5), and the like.

Next, in block 58, a standard modular stem member 12 is chosen from the set 26 according to the anatomy measured in block 56. More specifically, the stem member 12 is chosen so as to properly fit within the intramedularly canal and to attach to the existing cortical bone. More specifically, in some embodiments, the medical professional can choose a proper stem member 12 by looking at a longitudinal cross section of the femur 55. Then, a model of the smallest stem member 12 within the set 26 can be loaded into the model from the database 34. If that stem member 12 is too small to properly attach to the existing cortical bone, the medical professional can replace that stem with a progressively larger stem member 12 within the set 26 until a stem member 12 is identified that is large enough to properly couple to the femur 55.

Next, in block 60, the tool 32 is used to select an appropriate resection line R on the femur 55 based on the chosen stem member 12. Cut guides for the resection can also be generated in block 60.

Additionally, in block 61, a standard modular head member 14 is chosen from the set 22 according to the anatomy measured in block 56. Subsequently, in block 62, a standard modular neck member 16 is chosen from the set 22 to substantially match the anatomical parameters measured and identified in block 56.

Accordingly, the tool 32 loads virtual representations of one or more of the neck members 16a, 16b, 16c into the model and displays those neck members 16a, 16b, 16c on the display 40. In the embodiments represented in FIGS. 5, 6, and 7, the neck members 16a, 16b, 16c are illustrated within the model, shown in relation to the selected stem member 12 and head member 14, and shown overlaying the femur 55.

As shown in FIGS. 5, 6, and 7, each of the neck members 16a, 16b, 16c would provide either a different neck length Lₙ, neck angle α, amount of anteversion αₐₙₜ, or vector Dₐ if chosen for the prosthesis 20. In block 62, the neck member 16a, 16b, 16c in the set 22 that substantially matches the existing anatomy of the patient identified in block 56 is initially chosen. Thus, the neck member 16a, 16b, 16c that causes the prosthesis 20 to most closely match the existing anatomical parameters identified in block 56 is chosen for further analysis as will be described in greater detail.

In the embodiment shown, for instance, the neck member 16a might be selected in block 62, since it most closely matches the existing anatomy of the patient and the center C of the head member 14 most closely matches the center C' of the head 57 of the femur 55.

It will be appreciated that the set 24 may not include a neck member 16a, 16b, 16c that matches the existing anatomy exactly. Thus, the tool 32 can be adapted to choose the one neck member 16a, 16b, 16c that most closely matches each of the anatomical parameters Lₙ, α, αₐₙₜ, Dₐ within a predetermined threshold. Furthermore, the chosen neck member 16a, 16b, 16c may substantially match only some of the parameters Lₙ, α, αₐₙₜ, Dₐ. Thus, the degree of similarity can be expressed as a percentage covering each of the parameters.

Also, in block 63, the differences between the selected prosthesis 20 and the existing anatomy of the patient are identified and recorded. For instance, the tool 32 can detect the amount of difference between the anatomical parameters identified in block 56 and the prosthesis 20 that would result from the chosen neck member 16a. This difference can be illustrated on the display 40 to show the differences between the anatomy of the patient and the prosthesis 20.

Next, in decision block 64, it is determined whether the chosen neck member 16a provides adequate range of motion and stability when coupled with the selected head and stem members 12, 14. More specifically, using the computerized model, a hip joint mechanical analysis is performed using the selected neck member 16a, stem member 12, and head member 14. The hip joint mechanical analysis can be of any suitable type, such as a dynamic analysis of the hip joint using the selected neck member 16a, the selected stem member 12, and a selected head member 14 (FIG. 5).

More specifically, the medical professional can use the tool 32 in a range of motion analysis to determine the degree of movement available (i.e., to determine how much the prosthesis 20 can rotatably and linearly move relative to the pelvis without interference) using the selected neck member 16a, the selected stem member 12, and the selected head member 14. Also, the medical professional can use the tool 32 in a stability analysis to move the prosthesis 20 relative to the pelvis through common modes of dislocation (e.g., crossing the leg) and will determine if there is impingement between the components of the prosthesis 20, between bones, and/or between bones and the prosthesis 20. These analyses can be performed by moving the components virtually within the model.

Thus, in decision block 64, it is determined whether the range of motion provided by the prosthesis 20 is adequate and whether the prosthesis 20 provides sufficient stability over that identified range of motion. In some embodiments, the tool 32 is adapted to determine whether the range of motion is within a predetermined threshold and/or whether the stability is within a predetermined threshold.

If the selected neck member 16a provides sufficient stability and range of motion, it is determined that the chosen neck member 16a will be used in the prosthesis 20. Then, the method 50 continues in block 66.

In block 66, a trial provisional neck member (i.e., a prototype neck member intended for trial and not for implanting) is obtained. The provisional neck member corresponds in shape to the selected neck member 16a. In some embodiments, each of the neck members 16a, 16b, 16c in the set 24 includes a corresponding pre-made provisional neck member, and thus the surgeon can simply obtain the provisional neck member from the set 24. In other embodiments, the provisional neck member is made via a rapid prototyping machine once the neck member 16a is selected for implantation. It will be appreciated that the provisional neck member can be used by the surgeon during surgery, but before implanting the prosthesis 20, to confirm that the selected neck member 16a is adequate for the patient.

If decision block 64 is answered in the negative (i.e., the selected neck member 16a provides insufficient stability and/or range of motion), block 67 follows, and the medical professional uses the tool 32 to select another neck member 16b, 16c from the set 24 for use in the prosthesis 20. More specifically, if decision block 64 is answered in the negative, it is likely that the existing anatomy of the patient should not be replicated by using the neck member 16a selected in block 62. This could be due to deterioration, deformity, or other condition of the existing hip joint. Accordingly, it is determined that the neck member 16a should not be included in the prosthesis 20, and an alternative neck member 16b, 16c is selected in block 67. Thus, in block 67, the medical professional uses the tool 32 and his or her knowledge and expertise to select an alternative neck member 16b, 16c which will provide the prosthesis 20 with geometry that is different from the existing anatomy of the patient.

Then, in block 68, the tool 32 determines the adjusted geometry center of rotation of the head member 14 and the adjusted geometry of the prosthesis 20 using the alternate neck member 16b, 16c selected in block 67.

Next, in decision block 69, it is determined whether the alternate neck member 16b, 16c selected in block 67 provides adequate stability and range of motion. Decision block 69 is determined using the analyses described in detail above.

If the alternate neck member 16b, 16c provides adequate range of motion and stability (i.e., decision block 69 is answered affirmatively), the method 50 continues in block 66. In block 66, the prototype neck member is built.

However, if the alternate neck member 16 does not provide adequate stability and/or range of motion (i.e., decision block 69 is answered negatively), the method 50 continues in block 70, and custom designed member(s) (indicated at 30 in FIG. 2) are chosen for implantation as the prosthesis 20. As stated above, the prosthesis 20 could include a monolithic custom designed member 30 having a head, neck, and stem member, or the prosthesis 20 could include a custom designed neck member that is coupled with standard, modular head and stem members 14, 12. Then, a prototype of the custom designed members 30 are built in block 66.

In some embodiments, blocks 67, 68, and 69 are repeated for every alternate neck member 16b, 16c within the set 24. As such, a custom member 30 is chosen for the prosthesis 20 as a last resort.

Accordingly, the method 50 provides an accurate and efficient way of choosing the components of the patient matched prosthesis 20. The prosthesis 20 is substantially matched to the patient's needs to provide adequate stability and adequate range of motion, allowing the medical professional to replicate existing anatomy with the prosthesis 20 or to improve upon the existing anatomy. Furthermore, the method 50 allows the associated costs to be reduced by attempting to incorporate standard modular prosthetic components, or, as a last resort, incorporate one or more custom designed components.

Moreover, the foregoing discussion discloses and describes merely exemplary embodiments of the present disclosure. One skilled in the art will readily recognize from such discussion, and from the accompanying drawings and claims, that various changes, modifications and variations may be made therein without departing from the scope of the invention as defined by the following claims. For instance, the sequence of the blocks of the method described herein can be changed without departing from the scope of the present disclosure.

## Claims

1. A method of selecting a patient matched hip joint prosthesis (20) comprising:
pre-operatively imaging (52) a hip joint anatomy of a patient;
pre-operatively creating (54) an electronic model of the hip joint anatomy;
**characterised by**
pre-operatively identifying (56) on the electronic model a plurality of first parameters of the hip joint anatomy, the plurality of first parameters including a neck length of a femur (55) of the patient, a neck angle of the femur, an amount of anteversion of the femur, and a vector relative to a center of a head of the femur and an axis of a neck of the femur;
pre-operatively selecting (62) on the electronic model a standard modular neck member from a set of virtual representations of different standard modular neck members (16a, 16b, 16c) to substantially match the first parameters of the hip joint anatomy of the patient;
pre-operatively performing (64) on the electronic model a virtual hip joint mechanical analysis using the selected standard modular neck member;
pre-operatively choosing for implantation one of the selected standard modular neck member, a different one of the standard modular neck members (16a, 16b, 16c), and a custom neck member (30) based on the hip joint mechanical analysis; and
pre-operatively building (66) a provisional neck member corresponding in shape to the neck member chosen for implantation.

2. The method of claim 1, wherein each standard modular neck member (16a, 16b, 16c) in the set of standard modular neck members includes an end (27) adapted to removably couple to a stem member (12), and wherein the end (27) includes a male portion of a taper coupling.

3. The method of claim 1, further comprising assembling the patient matched hip joint prosthesis (20) from the custom designed neck member (30) and at least one of a standard, modular stem member (12) and a standard, modular head member (14).

4. The method of claim 1, wherein pre-operatively performing the hip joint mechanical analysis (64) comprises performing a dynamic analysis of the hip joint using the selected standard neck member.

5. The method of claim 1, wherein pre-operatively performing the hip joint mechanical analysis (64) comprises at least one of performing a range of motion analysis using the selected standard modular neck member and performing a stability analysis of the hip joint using the selected standard modular neck member.

6. The method of claim 1, wherein pre-operatively imaging (52) the hip joint includes imaging the hip joint anatomy by performing at least one of a CT scan, an MRI, and a fluoroscopic scan.

7. The method of claim 1, wherein at least one of the standard modular neck members (16c) includes a curved longitudinal axis.

8. The method of claim 7, further comprising determining whether the range of motion is within a predetermined threshold.

9. The method of claim 8, further comprising determining whether the stability is within a predetermined threshold.

10. The method of claim 1, further comprising displaying on the electronic model differences in the first parameters between the selected standard modular neck member and the anatomy of the patient.

11. The method of claim 8, further comprising determining any impingement between the selected standard neck member and the anatomy of the patient.

12. The method of claim 7, further comprising determining an amount of rotation and linear motion of the selected standard neck member without interference with the anatomy of the patient.

## Patentansprüche

1. Verfahren zum Auswählen einer an den Patienten angepassten Hüftgelenkprothese (20), umfassend:
präoperatives Abbilden (52) der Hüftgelenkanatomie eines Patienten;
präoperatives Erzeugen (54) eines elektronischen Modells der Hüftgelenkanatomie;
**gekennzeichnet durch**:
präoperatives Identifizieren (56) einer Anzahl erster Parameter der Hüftgelenkanatomie am elektronischen Modell, wobei die Anzahl erster Parameter die Halslänge eines Oberschenkels (55) des Patienten, den Halswinkel des Oberschenkels, das Ausmaß der Oberschenkel-Anteversion und einen Vektor relativ zur Mitte des Hüftkopfes und einer Achse eines Oberschenkelhalses beinhaltet;
präoperatives Auswählen (62) eines modularen Standard-Halsbauteils aus einem Satz virtueller Darstellungen verschiedener modularer Standard-Halsbauteile (16a, 16b, 16c) am elektronischen Modell, so dass es im Wesentlichen den ersten Parametern der Hüftgelenkanatomie des Patienten entspricht;
präoperatives Durchführen (64) einer virtuellen Hüftgelenk-Mechanikanalyse mit dem ausgewählten modularen Standard-Halsbauteil am elektronischen Modell;
präoperatives Auswählen eines ausgewählten modularen Standard-Halsbauteils, eines anderen modularen Standard-Halsbauteils (16a, 16b,
16c) und eines patientenspezifischen Halsbauteils (30) auf der Basis der mechanischen Hüftgelenk-Analyse zur Implantation; und
präoperatives Aufbauen (66) eines provisorischen Halsbauteils, dessen Form dem zur Implantation gewählten Halsbauteil entspricht.

2. Verfahren nach Anspruch 1, wobei jedes modulare Standard-Halsbauteil (16a, 16b, 16c) in dem Satz der modularen Standard-Halsbauteile ein Ende (27) umfasst, das lösbar an ein Stielbauteil (12) gekoppelt werden kann, und wobei das Ende (27) ein Steckteil einer Konus-Kopplung aufweist.

3. Verfahren nach Anspruch 1, zudem umfassend das Zusammenbauen der an den Patienten angepassten Hüftgelenkprothese (20) aus dem patientenspezifischen Halsbauteil (30) und mindestens einem modularen Standard-Stielbauteil (12) bzw. modularen Standard-Kopfbauteil (14).

4. Verfahren nach Anspruch 1, wobei bei dem präoperativen Durchführen der mechanischen Hüftgelenk-Analyse (64) eine dynamische Hüftgelenk-Analyse mit dem ausgewählten Standard-Halsbauteil durchgeführt wird.

5. Verfahren nach Anspruch 1, wobei bei dem präoperativen Durchführen der mechanischen Hüftgelenk-Analyse (64) mindestens ein Bereich der Bewegungsanalyse mit dem ausgewählten modularen Standard-Halsbauteil durchgeführt wird bzw. eine Stabilitätsanalyse des Hüftgelenks mit dem ausgewählten modularen Standard-Halsbauteil durchgeführt wird.

6. Verfahren nach Anspruch 1, wobei das präoperative Abbilden (52) des Hüftgelenks das Abbilden der Hüftgelenkanatomie beinhaltet, indem mindestens ein CT-Scan, eine MRI bzw. ein fluoroskopischer Scan durchgeführt wird.

7. Verfahren nach Anspruch 1, wobei mindestens eines der modularen Standard-Halsbauteile (16c) eine gekrümmte Längsachse beinhaltet.

8. Verfahren nach Anspruch 7, bei dem zudem bestimmt wird, ob der Bewegungsbereich innerhalb einer festgelegten Schwelle ist.

9. Verfahren nach Anspruch 8, bei dem zudem bestimmt wird, ob die Stabilität innerhalb einer festgelegten Schwelle ist.

10. Verfahren nach Anspruch 1, bei dem zudem auf dem elektronischen Modell Unterschiede in den ersten Parametern zwischen dem ausgewählten modularen Standard-Halsbauteil und der Anatomie des Patienten angezeigt werden.

11. Verfahren nach Anspruch 8, bei dem zudem jeglicher Zusammenstoß zwischen dem ausgewählten Standard-Halsbauteil und der Anatomie des Patienten bestimmt wird.

12. Verfahren nach Anspruch 7, bei dem zudem das Rotationsausmaß und die lineare Bewegung des ausgewählten Standard-Halsbauteils ohne Störung der Anatomie des Patienten bestimmt werden.

## Revendications

1. Procédé de sélection d'une prothèse d'articulation de la hanche adaptée au patient (20) comprenant :
l'imagerie préopératoire (52) d'une anatomie de l'articulation de la hanche d'un patient ;
la création préopératoire (54) d'un modèle électronique de l'anatomie de l'articulation de la hanche ;
**caractérisé par**
l'identification préopératoire (56) sur le modèle électronique d'une pluralité de premiers paramètres de l'anatomie de l'articulation de la hanche, la pluralité de premiers paramètres incluant une longueur du col d'un fémur (55) du patient, un angle de col du fémur, une quantité d'antéversion du fémur et un vecteur relatif à un centre d'une tête du fémur et un axe d'un col du fémur ;
sélectionner de manière préopératoire (62) sur le modèle électronique un élément de col modulaire standard à partir d'un ensemble de représentations virtuelles de différents éléments de col modulaires standard (16a, 16b, 16c) afin de correspondre aux premiers paramètres de l'anatomie de l'articulation de la hanche du patient ;
exécuter de manière préopératoire (64) sur le modèle électronique une analyse mécanique d'articulation de la hanche virtuelle en utilisant l'élément de col modulaire standard sélectionné ;
sélectionner de manière préopératoire pour l'implantation un parmi l'élément de col modulaire standard sélectionné, un différent des éléments de col modulaires standard (16a, 16b, 16c), et un élément de col particulier (30) basé sur l'analyse mécanique d'articulation de la hanche ; et
construire de manière préopératoire (66) un élément de col provisoire dont la forme correspond à l'élément de col sélectionné pour l'implantation.

2. Procédé selon la revendication 1, dans lequel chaque élément de col modulaire standard (16a, 16b, 16c) dans l'ensemble des éléments de col modulaires standard comprend une extrémité (27) apte à être couplée d'une manière amovible à un élément de tige (12), et où l'extrémité (27) comprend une portion male d'un couplage conique.

3. Procédé selon la revendication 1, comprenant en outre l'assemblage de la prothèse d'articulation de la hanche adaptée au patient (20) à partir de l'élément de col conçu particulier (30) et au moins un parmi un élément de tige standard, modulaire (12) et un élément de tête modulaire standard (14).

4. Procédé selon la revendication 1, dans lequel l'exécution préopératoire de l'analyse mécanique de l'articulation de la hanche (64) comprend l'exécution d'une analyse dynamique de l'articulation de la hanche en utilisant l'élément de col standard sélectionné.

5. Procédé selon la revendication 1, dans lequel l'exécution préopératoire de l'analyse mécanique de l'articulation de la hanche (64) comprend au moins une parmi l'exécution d'une plage d'analyse de mouvements en utilisant l'élément de col modulaire standard sélectionné et l'exécution d'une analyse de stabilité de l'articulation de la hanche en utilisant l'élément de col modulaire standard sélectionné.

6. Procédé selon la revendication 1, dans lequel l'imagerie préopératoire (52) de l'articulation de la hanche comprend l'imagerie d'une anatomie de l'articulation de la hanche en exécutant au moins un parmi un scan CT, une IRM et un scan fluoroscopique.

7. Procédé selon la revendication 1, dans lequel au moins un des éléments de col modulaires standard (16c) comprend un axe longitudinal courbé.

8. Procédé selon la revendication 7, comprenant en outre la détermination si la plage de mouvements est dans un seuil prédéterminé.

9. Procédé selon la revendication 8, comprenant en outre la détermination si la stabilité est dans un seuil prédéterminé.

10. Procédé selon la revendication 1, comprenant en outre l'affichage sur le modèle électronique des différences dans les premiers paramètres entre l'élément de col modulaire standard sélectionné et l'anatomie du patient.

11. Procédé selon la revendication 8, comprenant en outre la détermination de tout impact entre l'élément de col standard sélectionné et l'anatomie du patient.

12. Procédé selon la revendication 7, comprenant en outre la détermination d'une quantité de rotation et d'un mouvement linéaire de l'élément de col standard sélectionné sans interférence avec l'anatomie du patient.
